# EUROPEAN PATENT SPECIFICATION

(11) **EP 0 522 086 B1**
(45) Date of publication and mention of the grant of the patent: **20.07.1994**
(21) Application number: 91908143.0
(22) Date of filing: 08.03.1991
(51) Int. Cl.: C12N 15/12, G01N 33/574, C12Q 1/68, C12N 5/10, C12P 21/08

(54) **DNA SEQUENCE ENCODING GALACTOPROTEIN b3**
DNS-SEQUENZ, DIE FÜR GALACTOPROTEIN B3 KODIERT
SEQUENCE CODANT LA GALACTOPROTEINE b3

(30) Priority: 12.03.1990 US 491910
(43) Date of publication of application: 13.01.1993
(73) Proprietor: The Biomembrane Institute, Seattle Washington 98119 (US)
(72) Inventor: TSUJI, Tsutomu, Nerima-ku, Tokyo 179 (JP); YAMAMOTO, Fumi-ichiro, Bellevue, WA 98006 (US); HAKOMORI, Sen-itiroh, Mercer Island, WA (US)
(74) Representative: Brown, John David
(86) International application number: US9101606
(87) International publication number: WO9113983

(56) References cited:
- EP-A- 0 344 134
- The Journal of Biological Chemistry, vol. 265, no. 12, 25 April 1990, The American Society for Biochemistry and Molecular Biology, Inc., (Baltimore, US), T. Tsuji et al.
- Biochemical and Biophisical Research Communications, vol. 76, no. 2, 1977, Academic Press, Inc., (Duluth, Minnesota, US), W.G. Carter et al.
- Proc. Natl. Acad. Sci. USA, vol. 85, no. 11, June 1988, (Washington,DC, US), J. Viitala et al.

## Description

### Technical Field

The present invention relates to the glycoprotein galactoprotein b3 and its uses. This invention is more particularly related to the galactoprotein b3 gene, probes to the DNA sequences, methods for tumor detection, DNA constructs, recombinant plasmids, recombinant methods for producing galactoprotein b3, purified galactoprotein b3 and antibodies produced therefrom.

### Background of the Invention

Despite enormous investments of financial and human resources, cancer remains one of the major causes of death. Early diagnosis is essential to prevent radical surgery or loss of life.

In order to detect or treat any form of cancer, it is necessary to be able to distinguish the transformed cells from the normal cells. Comparisons of normal and cancer cells have focused on transformation-dependent changes in the molecules residing at the surface of cell membranes. These molecules include glycolipids, proteins and glycoproteins. Certain molecules have been found to disappear or at least decrease greatly upon oncogenic transformation, while other molecules increase.

Galactoprotein a, later renamed as fibronectin, is an example of a major glycoprotein that decreases upon oncogenic transformation. Fibronectin has been extensively characterized. Conversely, although the expression of galactoprotein b3 ("Gap b3") was found to be enhanced at the surface of polyoma-transformed NIL cells as compared to progenitor NIL cells, there has been very little study of Gap b3.

Consequently, because of the association between an increased expression of Gap b3 and oncogenic transformation, there exists a need in the art for the primary structure of the gene encoding Gap b3. The present invention fills this need and further provides other related advantages.

### Summary of the Invention

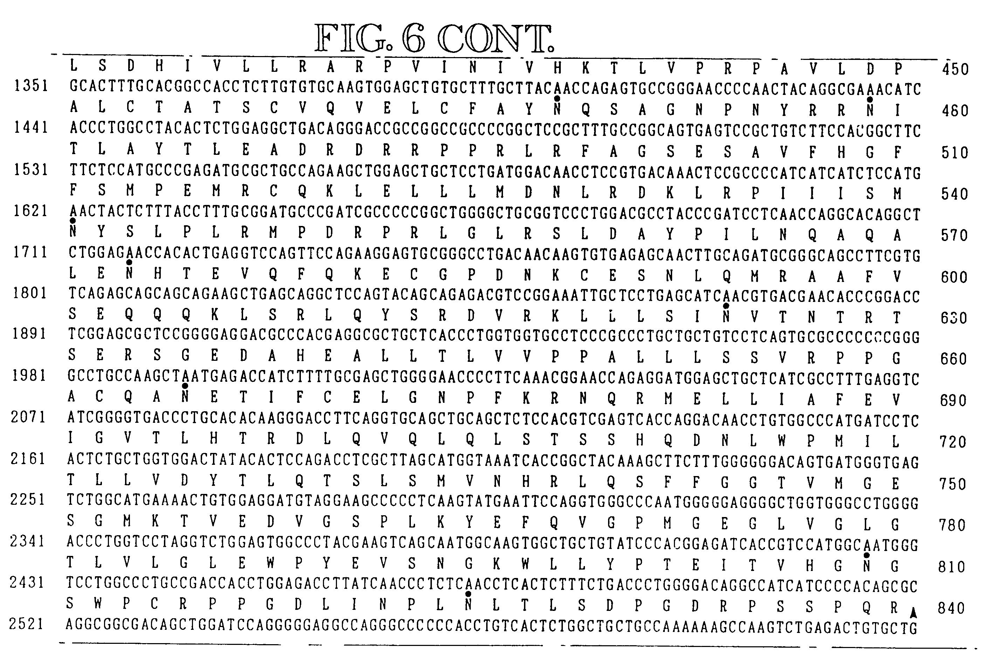

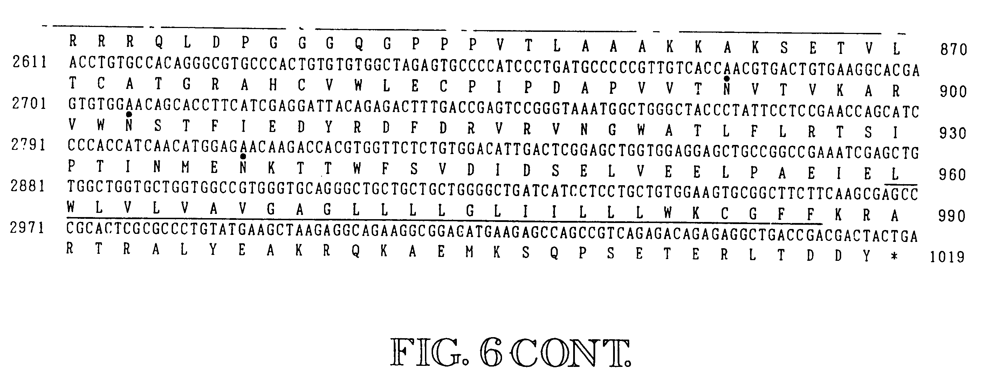

Briefly stated, the present invention provides an isolated DNA molecule encoding galactoprotein b3 said protein obtainable from hamster fibroblast NIL cells transformed with polyoma virus and said protein having an approximate molecular weight of 140,000 as determined by sodium dodecyl sulfate-polyacrylamide gel electrophoresis under non-reducing conditions. Within one embodiment, the DNA molecule encodes human galactoprotein b3, said protein obtainable from hamster fibroblast NIL cells transformed with polyoma virus and said protein having an approximate molecular weight of 140,000 as determined by sodium dodecyl sulfate-polyacrylamide gel electrophoresis under non-reducing conditions. Within another embodiment, the DNA molecule encodes galactoprotein b3 comprising the amino acid sequence shown in Figure 6 from phenylalanine, amino acid 1, to tyrosine, amino acid 1019. Also disclosed is an isolated DNA molecule capable of specifically hybridizing with a DNA molecule encoding galactoprotein b3. The present invention further discloses a DNA construct comprising a DNA sequence encoding galactoprotein b3.

In another aspect of the present invention, recombinant plasmids capable of expression in a host cell, the recombinant plasmid further comprising a DNA sequence encoding galactoprotein b3 are provided. Suitable promoters and/or polyadenylation signals are also disclosed. In addition, eukaryotic cells transfected with recombinant plasmids comprising a DNA sequence encoding galactoprotein b3, and methods for producing galactoprotein b3 using host cells transfected or transformed with a suitable DNA molecule are also disclosed. A method for producing galactoprotein b3 comprises: introducing into a eukaryotic host cell a DNA sequence encoding galactoprotein b3; growing the host cell in an appropriate medium; and isolating the protein product, encoded by the DNA sequence, produced by the host cell.

Within a related aspect, the present invention discloses antibodies that specifically bind to galactoprotein b3. Preferred antibodies include monoclonal antibodies.

In another aspect of the present invention, methods are provided for the detection of a tumor that results in the expression of galactoprotein b3 at levels exceeding that expressed by non-transformed cells. Within one embodiment, the method comprises the steps of:
(a) measuring the level of RNA specific for galactoprotein b3 in RNA isolated from a warm-blooded animal;
(b) measuring the level of RNA specific for galactoprotein b3 in control RNA isolated from nontransformed, galactoprotein b3-expressing cells from a warm-blooded animal; and
(c) comparing the levels of RNA measured in steps (a) and (b), and thereby determining the presence or absence of the tumor.

Within another embodiment, the method comprises the steps of:
(a) incubating a biological sample from a warm-blooded animal, said sample suspected of containing galactoprotein b3, with an antibody that specifically binds to galactoprotein b3 under conditions and for a time sufficient to allow immunocomplexes to form therebetween;
(b) detecting immunocomplexes formed between said antibody and galactoprotein b3 in said biological sample;
(c) incubating a control biological sample, wherein said control sample is from a warm-blooded animal possessing non-transformed cells expressing galactoprotein b3, with an antibody that specifically binds to galactoprotein b3 under conditions and for a time sufficient to allow immunocomplexes to form therebetween;
(d) detecting immunocomplexes formed between said antibody and galactoprotein b3 in said control biological sample; and
(e) comparing the number of immunocomplexes detected in steps (b) and (d), and thereby determining the presence or absence of the tumor.

These and other aspects of the present invention will become evident upon reference to the following detailed description and attached drawings.

### Brief Description of the Drawings

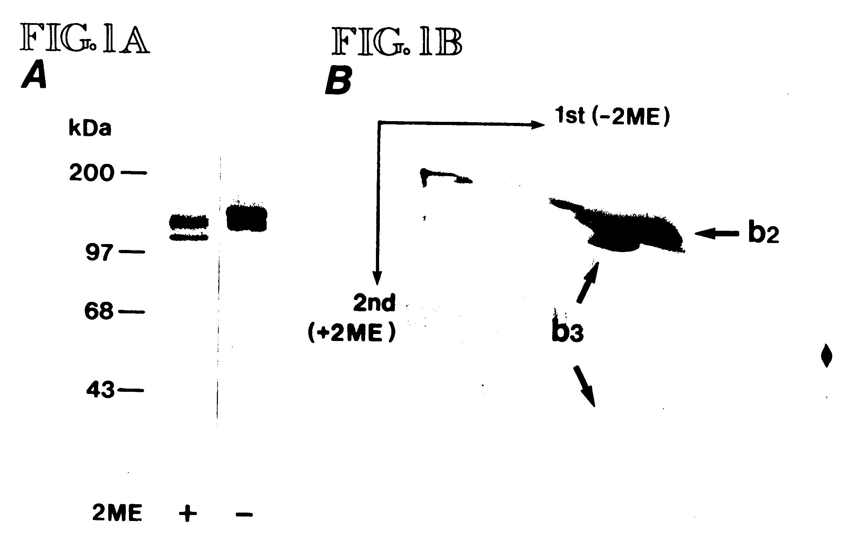

Figure 1 shows the separation of Gap b proteins by SDS-PAGE. Gap b fraction from ³H-labeled NILpy cells was separated by 1-D (A) and 2-D (B) SDS-PAGE in the presence (+) and absence (-) of 2-mercaptoethanol (2-ME). Cell surface labeling was performed by galactose oxidase/NaB³H₄ method. Positions of protein molecular weight standards are indicated. Eight percent and 12.5% polyacrylamide gel were used for 1st and 2nd dimension, respectively, in 2-D gel analysis. Gap b3 (b3) was found to split into two spots, whereas Gap b2 (b2) remained as a single spot in the 2-D gel.

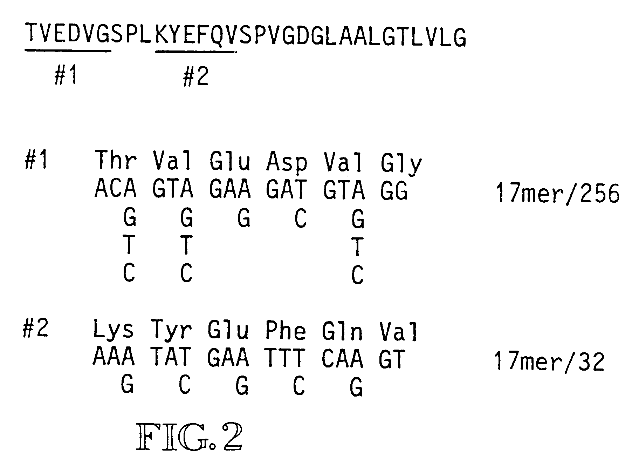

Figure 2 depicts a partial amino acid (a.a.) sequence of an internal peptide and corresponding degenerate oligodeoxynucleotide sequences used as probes. Gap b3 heavy chain was digested with Achromobacter protease I, and the resulting peptides were separated by reversed-phase HPLC. The sequence is shown in the top panel. The a.a. sequences of two portions of one peptide were used for synthesis of oligonucleotide probes. Nucleotide sequences for oligos (#1 and #2) are shown in the bottom panel. Their degeneracies were 256 and 32, respectively.

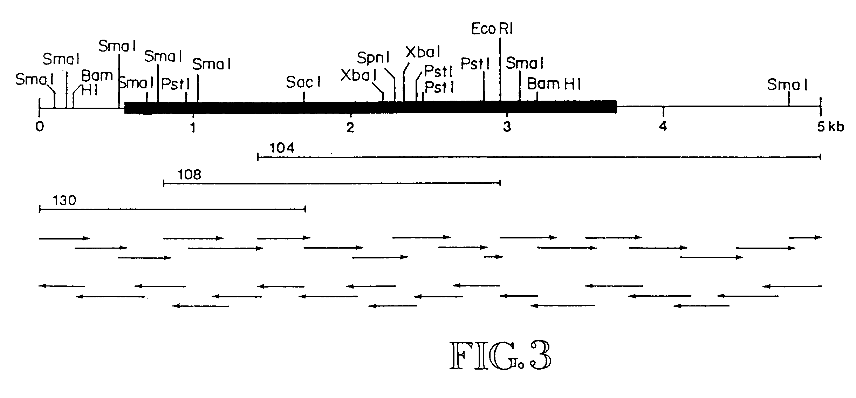

Figure 3 illustrates the restriction map for three overlapping clones encoding hamster Gap b3 and sequencing strategy. Complete cDNA was cloned in three overlapping clones (104, 108, and 130) to cover approximately 5 kb. Protein coding and untranslated regions are shown by thick and thin lines, respectively. The arrows indicate the direction and extent of the individual sequencings.

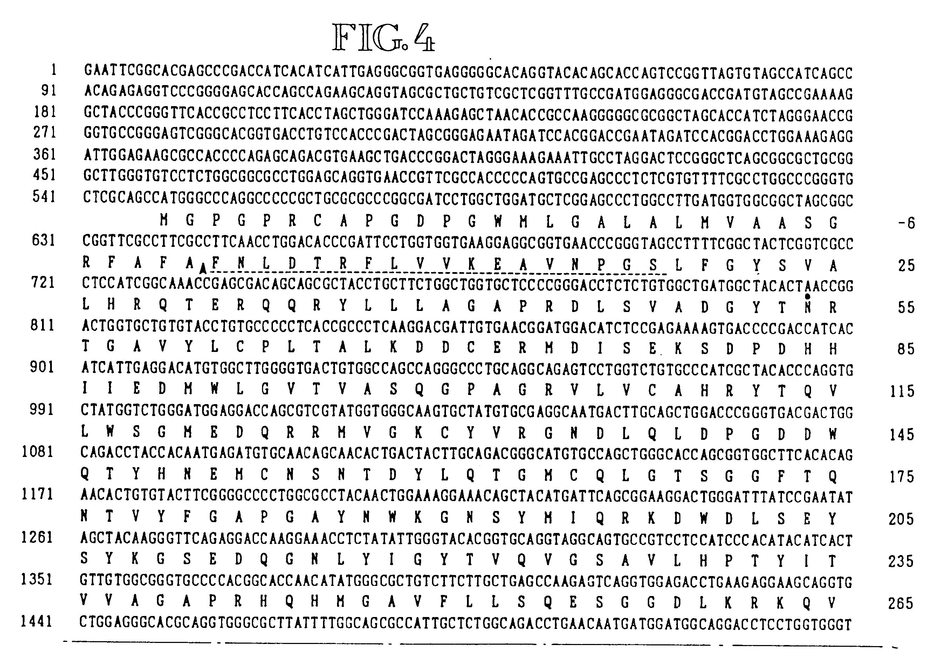

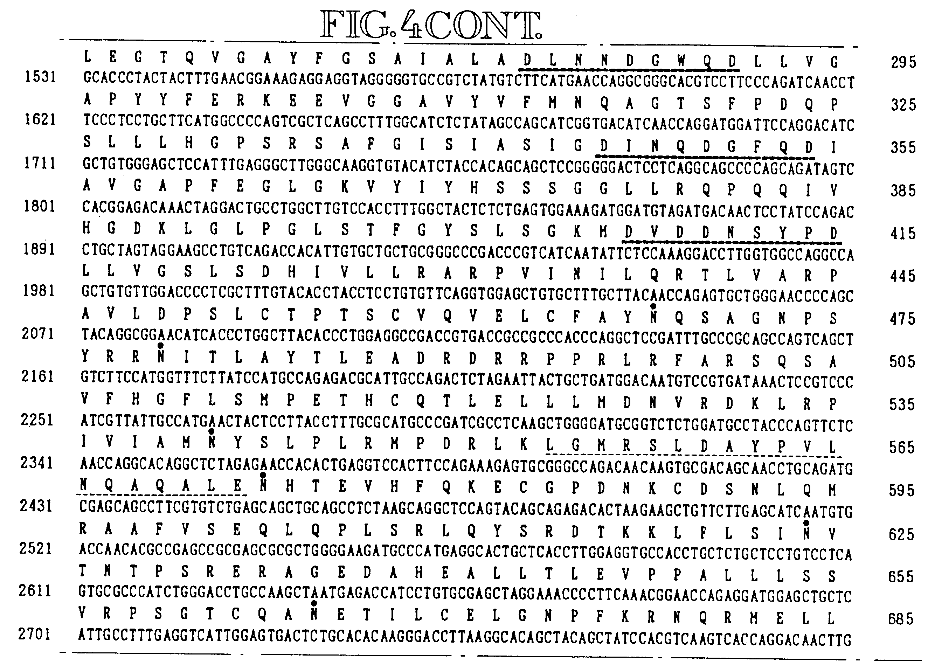

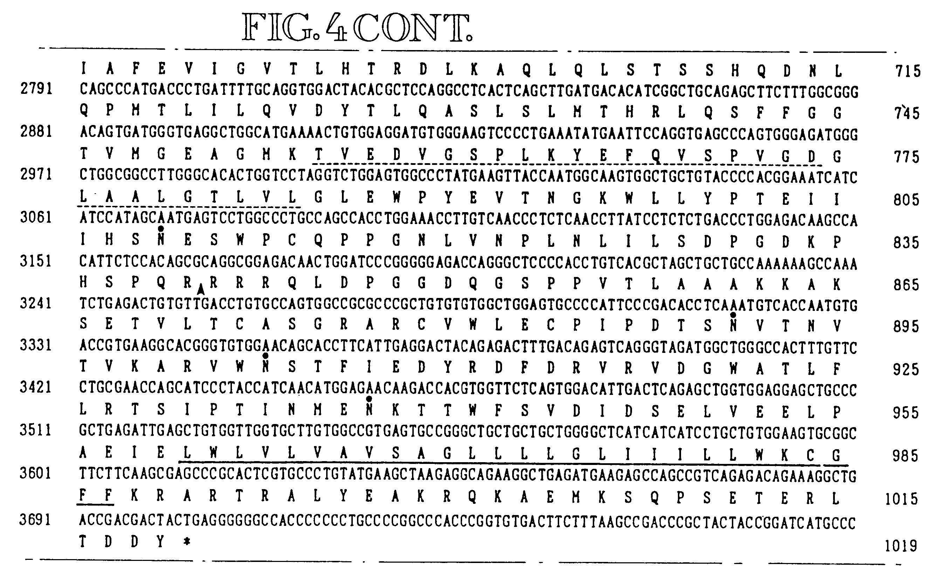

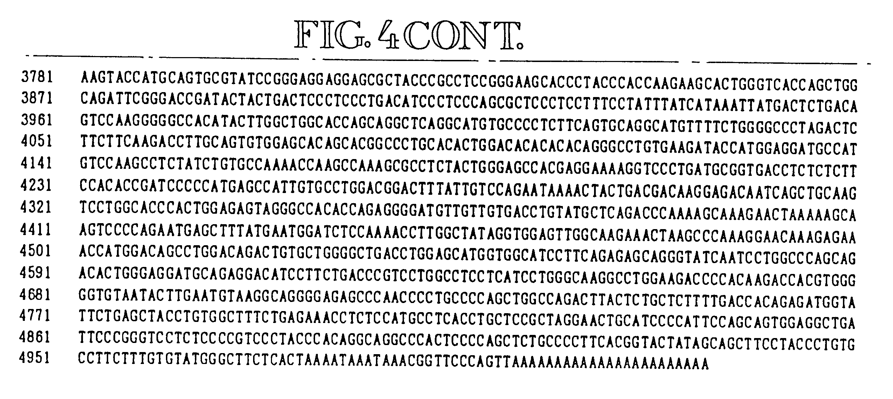

Figure 4 depicts the nucleotide and deduced amino acid sequence of hamster Gap b3. Nucleotide and deduced a.a. sequence of the entire cDNA of Gap b3 is shown. The N-terminal a.a. of the purified Gap b3 (F) is numbered 1. Thirty-two a.a. residues precede this N-terminus. Symbols: thin broken underlines, matching positions of a.a. sequences determined by sequence analysis; solid underline, putative transmembrane domain; dots, potential Asn-linked glycosylation sites (N X S/T); arrows, possible cleavage sites; thick broken underline, putative divalent cation-binding sites. Polyadenylation signals are found at nucleotide positions 4979 and 4983.

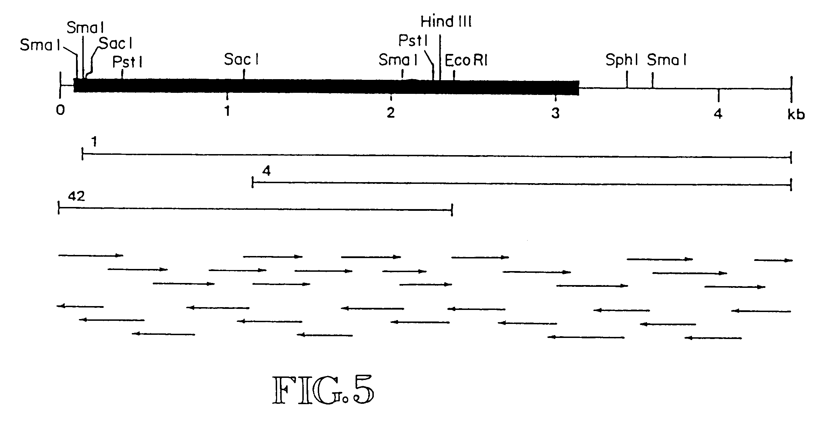

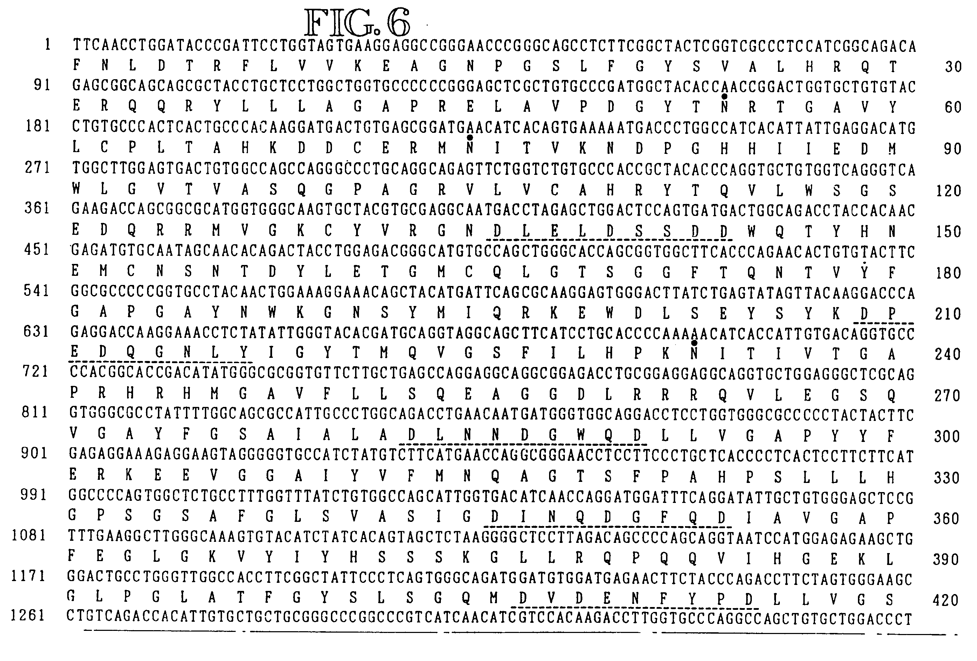

Figure 5 illustrates the restriction map for three overlapping clones encoding human Gap b3 and sequencing strategy. The coding region is shown by a thick line. The arrows indicate the direction and extent of the individual sequencings.

Figure 6 depicts the nucleotide and deduced amino acid sequence of the coding region of human Gap b3. The N-terminal a.a. of the mature human Gap b3 polypeptide (F) is numbered 1. Symbols: underline, putative transmembrane domain; dots, potential Asn-linked glycosylation sites (NXS/T); arrow, possible cleavage site; broken underline, putative divalent cation-binding sequences. Three typical sequences (located on C-terminal side) and two related sequences (located on N-terminal side) are shown.

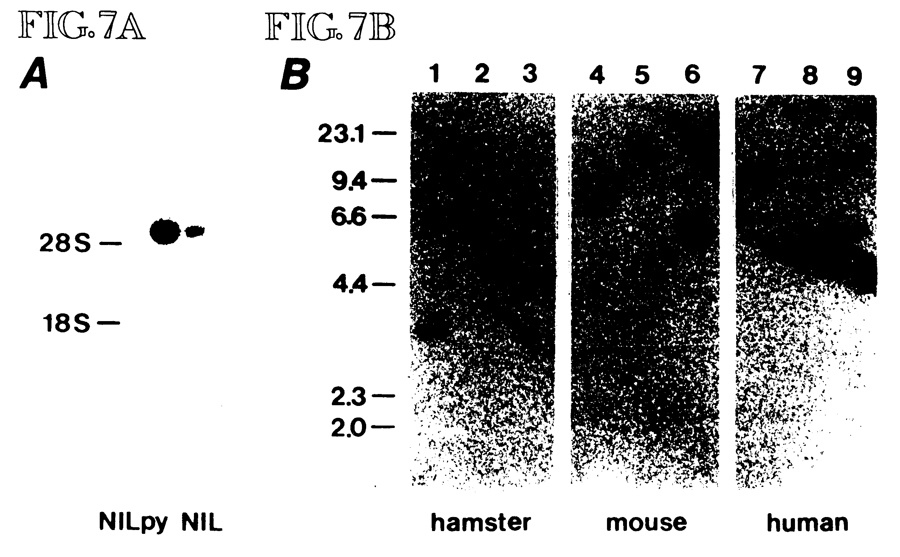

Figure 7 shows Northern and Southern hybridization analysis. In part A, poly A⁺ RNA (5 µg) isolated from NILpy (left) and NIL (right) was electrophoresed on a 1% formaldehyde-agarose gel. Positions of rRNAs (28S, 18S) are indicated. In part B, genomic DNAs (10 µg) from hamster (lanes 1-3), mouse (4-6), and human (7-9) were digested with Eco RI (lanes 1, 4, 7), Hind III (2, 5, 8), or BamHI (3, 6, 9), and electrophoresed on a 1% agarose gel. Both Northern and Southern blots were probed with a ³²P-labeled 400 bp Pst I fragment of a hamster Gap b3 clone. Positions of marker (lambda digested with Hind III) are indicated.

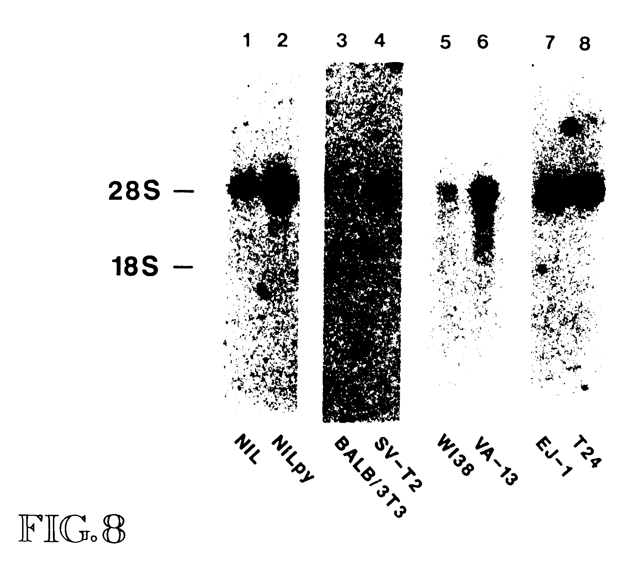

Figure 8 shows Northern hybridization analysis. Poly A⁺ RNA (4 µg), isolated from various cell lines, was electrophoresed on a 1% formaldehyde-agarose gel. Membrane filters were probed with a ³²P-labeled 400-bp PstI fragment of a hamster Gap b3 clone. Lanes 1 and 2, hamster fibroblast NIL and its polyoma virus transformant NILpy; lanes 3 and 4, mouse fibroblast BALB/3T3 and its SV-40 transformant SV-T2; lanes 5 and 6, human fibroblast WI-38 and its SV-40 transformant WI-38/VA-13; lanes 7 and 8, human bladder carcinoma cell lines EJ-1 and T24. Positions of rRNAs (28S, 18S) are indicated.

### Detailed Description of the Invention

Prior to setting forth the invention, it may be helpful to an understanding thereof to set forth definitions of certain terms to be used herein.

Antibody - as used herein, includes an intact molecule, a fragment thereof, or a functional equivalent thereof; and may be genetically engineered. Examples of antibody fragments include F(ab')₂, Fab', Fab and Fv.

Complementary DNA or cDNA - a DNA molecule or sequence which has been enzymatically synthesized from the sequences present in an mRNA template, or a clone of such a molecule.

DNA Construct - a DNA molecule, or a clone of such a molecule, either single- or double-stranded, which has been modified to contain segments of DNA which are combined and juxtaposed in a manner which would not otherwise exist in nature.

Plasmid or Vector - a DNA sequence containing genetic information which may provide for its replication when inserted into a host cell. A plasmid generally contains at least one gene sequence to be expressed in the host cell, as well as sequences which facilitate such gene expression, including promoters and transcription initiation sites. It may be a linear or closed circular molecule.

The present invention provides the glycoprotein galactoprotein b3. This protein (hereinafter referred to as "Gap b3") is a membrane-bound protein whose expression increases on oncogenic transformation.

Gap b3 may be isolated by a combination of extraction and chromatography techniques. Briefly, in one embodiment, Gap b3 is extracted from mammalian cells by homogenization and solubilization with detergent. The detergent extract is passed over a lectin affinity chromatography column. The fraction containing Gap b3 is eluted using a solution containing a specific sugar, such as lactose. Final purification is performed using preparative sodium dodecyl sulfate polyacrylamide gel electrophoresis (SDS-PAGE).

A variety of mammalian cells are suitable for purification of Gap b3. A preferred source of starting material for such purification is oncogenically-transformed hamster fibroblasts. A representative isolation procedure is as follows. Homogenization of cells in a buffer solution containing a detergent such as Empigen BB yields a solution with galactoproteins b. The soluble supernatant of the extract may be adsorbed on RCA (Ricinus communis lectin) coupled to Sepharose and eluted with lactose. Final purification of Gap b3 to homogeneity is achieved by preparative SDS-PAGE chromatography, e.g., by application of a lactose eluate to a SDS slab gel.

A representative purified Gap b3 of the present invention has the following characteristics. Two major bands with apparent Mr 125,000 and 100,000 were observed in the eluate from a RCA-Sepharose column by 0.3 M lactose-containing buffer when analyzed on SDS-PAGE in the presence of 2-mercaptoethanol (2-ME) (Figure 1A). These bands are referred to hereinafter as galactoprotein b2 and b3 (Gap b2 and b3), respectively. In the absence of 2-ME, a different electrophoretic profile was observed (Figure 1A), in which the sharp band (Gap b3) was slightly shifted to the high Mr region, while the mobility of the broad band (Gap b2) was unchanged. The apparent Mr of Gap b3 under non-reducing conditions was estimated at 140,000. To confirm the change in mobility, 2-D gel electrophoresis was carried out in the absence (1st dimension) and presence (2nd dimension) of 2-ME. As shown in Figure 1B, Gap b3 was found to split into two spots (indicated by arrows as "b3"). Mobility of these spots in the 1st dimension was slightly lower than that of Gap b2, and was located between the Gap b1 and b2 spots.

Gap b3 appears to be composed of two disulfide-linked polypeptide chains with approximate Mr 110,000 and 30,000. The latter component was labeled very weakly by galactose oxidase/NaB³H₄. Gap b2 and the heavy chain of Gap b3 were separately purified and electrophoretically extracted from the gel fragments.

The present invention also provides antibodies that specifically bind to Gap b3, i.e., exhibit a binding affinity of about 10⁷ liters/mol. The antibodies are useful tools for the cytolocalization, e.g., by immuno-gold electron microscopy, of Gap b3 and for elucidating its role in cellular differentiation and malignant transformation. The purified Gap b3 protein described above may be utilized to produce polyclonal or monoclonal antibodies which bind to Gap b3. It will be evident to one skilled in the art that antibodies to fragments of Gap b3 may also be produced. Antibodies to intact, denatured Gap b3 are particularly useful for detection of "fixed," e.g., formaldehyde or glutaraldehyde, cells expressing Gap b3.

Briefly, polyclonal antibodies may be produced by immunization of an animal and subsequent collection of its sera. It is generally preferred to follow the initial immunization with one or more boosters prior to sera collection.

Monoclonal antibodies (MAbs) may be generally produced by the method of Kohler and Milstein (Nature 256:495-497, 1975; Eur. J. Immunol. 6:511-519, 1976). Briefly, the lymph nodes and/or spleens of an animal injected with purified protein are fused with myeloma cells to form hybrid cell lines ("hybridomas" or "clones"). Each hybridoma secretes a single type of immunoglobulin specific for the protein, and, like the myeloma cells, has the potential for indefinite cell division.

The MAbs of the present invention are produced by immunization of an animal with substantially pure Gap b3. Spleen cells are fused with myeloma cells and hybridomas cloned by limiting dilution procedures. Hybridomas may be selected on the basis of reactivity with the purified Gap b3 which is attached to a solid phase, staining of cells possessing high expression of Gap b3, and/or immunoprecipitation of labeled Gap b3.

The present invention also provides isolated DNA molecules, including genomic DNA and cDNA, encoding Gap b3. Based on the partial amino acid sequence of the heavy chain of Gap b3, the cDNA encoding hamster Gap b3 was cloned. The cloning strategy may be briefly summarized as follows: (1) synthesis of degenerate oligodeoxy-nucleotides reverse translated from amino acid sequence; (2) cDNA preparation; (3) cDNA library construction; and (4) screening the library with ³²P-labeled oligodeoxynucleotide probe. More specifically, for the isolation of a representative DNA molecule encoding a Gap b3, poly A+ RNA from the hamster polyoma-transformed NIL (NILpy) cell line (which expresses high levels of Gap b3) was used for construction of a λgt10 cDNA library. Alternatively, a cDNA library may be constructed from human tissue.

Degenerate synthetic oligodeoxynucleotide probes based on the partial amino acid sequences of Gap b3 were constructed as shown in Figure 2. cDNA was constructed by random-priming. Hybridization and washing temperatures were determined by the following formula: Tm = 4(G + C) + 2 (T + A). Specific hybridization with a DNA molecule encoding Gap b3 is achieved using the hybridization and washing conditions described below (Example 3E). Identity of the candidate clones was tested by the hybridization with oligo probes prepared for different regions of Gap b3. Screening of a hamster NILpy cDNA library (5x10⁵ independent phages) with the two probes shown in Figure 2 resulted in the isolation of 15 double positive clones. Clone #104 had the longest insert (3.7 kb) (Figure 3). None of them, however, was found to contain the N-terminal region of Gap b3. To obtain additional clones containing this region, the primer extension library was screened; one clone (#130) was found to have the longest insert containing this region. Based on mapping and partial sequence analysis, three clones (#104, 108, 130) were aligned as shown in Figure 3. Identity of these clones as cDNA for Gap b3 was confirmed by the presence of the N-terminal sequence of FNLDTRFLVVKEAVNPGS, and two internal sequences identified by the sequence analysis, i.e., TVEDVGSPLKYEFQVSPVGDGLAALGTLVLG and LGMRSLDAYPVLNQAQALE, which were identical to those deduced from the nucleotide sequence of cDNA (Figure 4).

The complete cDNA sequence and deduced amino acid (a.a.) sequence of hamster Gap b3 are shown in Figure 4. The whole cDNA sequence was constructed from the three overlapping partial cDNA clones. The deduced a.a. sequence contains the N-terminus and two other a.a. sequences obtained from protein sequence analysis of Gap b3 heavy chain. A relative hydrophobic region spanning 32 a.a. residues which precedes the N-terminal sequence (FNLDTR----) appears to be a signal sequence initiated by a methionine residue at the -32 position. The nucleotide sequence surrounding this methionine codon ATG (GCCATGG) was close to the consensus sequence (ACCATGG) for the initiation of translation, and identical to the one for murine lymph node homing receptor and for human N-acetylgalactosaminyl transferase. Two overlapping polyadenylation signals (AATAAATAAA) were found 22 nucleotides before the poly A tail.

In a similar manner, DNA molecules encoding human Gap b3 were isolated. Screening of a human T24 (bladder carcinoma cell line) cDNA library (> 2 kbp, 2 x 10⁵ independent phages) with a hamster Gap b3 cDNA probe resulted in the isolation of 20 positive clones. Clone no. 1 had the largest insert (4.4 kbp) (Figure 5). None of them, however, was found to contain the N-terminal region of the polypeptide. To obtain additional clones containing this region, the same library was screened with 5′-terminus of cDNA clone no. 1 (180 bp PstI fragment) as a probe; one clone (no. 42) was found to contain this region. Based on mapping and partial sequence analyses, three clones (nos. 1, 8, and 42) were aligned as shown in Figure 5.

The cDNA sequence and the deduced amino acid sequence in the coding region of human Gap b3 are shown in Figure 6. The cDNA sequence was constructed from three overlapping partial cDNA clones. Identity of these clones as cDNA for the human counterpart of Gap b3 was confirmed by the same number (1019) of amino acid residues as the authentic hamster Gap b3 and a quite high sequence homology (89% for the amino acids), including all 19 Cys residues at matched positions.

The mature polypeptide from either hamsters or humans contains 1019 a.a. residues and has a predicted Mr of 113,295 and 113,504, respectively. These values are in fairly good agreement with the estimated Mr of deglycosylated Gap b3 (115,000), which was prepared by glycopeptidase F (Boehringer Mannheim Biochemicals, Indianapolis, Ind.) digestion. The hamster sequence contains 11 potential N-glycosylation sites (Asn-X-Ser/Thr, where X is not Pro), and the human sequence contains 14 such sites (Figures 4 and 6, respectively). If a carbohydrate chain with an average Mr of 2,500 is assumed to attach to 11 such moieties on hamster Gap b3 or 14 such moieties on human Gap b3, the total Mr would be 140,795 or 148,504, respectively. The former value is also consistent with the estimated Mr of 140,000 from the relative mobility of hamster Gap b3 on SDS-PAGE under non-reducing conditions, as described above. Since Gap b3 appears to be composed of two disulfide-linked polypeptide chains, a single mRNA may encode a single polypeptide chain which is cleaved into the two chains by proteolysis after translation.

Based upon hydrophobicity plot analysis, a hydrophobic region consisting of a stretch of 28 a.a. residues is located near the C-terminus of both hamster and human Gap b3. This hydrophobic a.a.-clustered region presumably is a transmembrane domain. Since all 11 (for hamster) or 14 (for human) potential carbohydrate attachment sites are located on the N-terminal side of the putative transmembrane domain, and no such site is on the C-terminal side, Gap b3 appears to be oriented in the membrane with its N-terminus outside the cell. The C-terminal 32-a.a. segment is, therefore, likely to constitute the cytoplasmic domain. The long segment comprised of the remaining 959 a.a. residues (94%) is presumably an extracellular domain.

At about one third of the distance from the N-terminus of both hamster and human Gap b3 is a three homologous sequence motif with the consensus sequence D X D/N X D/N G X X D (Figures 4 and 6, respectively). This sequence is usually responsible for metal binding, e.g., Ca²⁺ and Mg²⁺. The N-terminal region of the human polypeptide has seven homologous repeats, three of which include the putative metal binding sequences. Each repeat consists of a long (21-28 amino acids) and a following short (five amino acids) stretch. The alignment of glycine and hydrophobic amino acid residues in each repeat shows a similar pattern, especially the presence of XXXGAP sequence (where X is a hydrophobic aminio acid) at the end of most of the longer stretches (except for the second and seventh repeats).

As noted above, Gap b3 is a transformation-dependent cell surface glycoprotein. mRNA levels for the glycoprotein before and after transformation were compared by Northern blot analysis. Figure 7A shows that a transcript of ≈5.0 kb was identified in RNA from both NILpy and NIL cells. Furthermore, mRNA for Gap b3 appeared much more abundant in the transformed cells than in the progenitor cells. Hybridization of the same blot with a cDNA probe for the β-actin gene gave comparable signals in the two lanes. These results indicate that enhanced expression of Gap b3 is transcriptionally controlled by its mRNA level. Similarly, Northern blot analysis was carried out on RNA from murine and human fibroblasts before and after oncogenic transformation induced by SV-40. Figure 8 shows that fibroblasts of human (WI-38) and mouse (BALB/3T3), and their respective SV-40 transformants, gave a single transcript (-4.8 kb) hybridized with the hamster-derived Gap b3 cDNA probe. Furthermore, SV-40 transformed cells were found to contain larger amounts of mRNA than the progenitor cells from both human and mouse as well as hamster. Hybridization of the same blot with a cDNA probe for the β-actin gene gave comparable signals in the six lanes. The increase of mRNA for Gap b3 after the transformation is a general phenomenon among human, mouse, and hamster fibroblasts. Figure 8 also shows that two human bladder carcinoma cell lines (EJ-1 and T24) contain high levels of mRNA for Gap b3.

Southern hybridization was performed to analyze DNAs from different species of mammals. Southern blot analysis of genomic DNA digested with three different endonucleases showed that each digested DNA from all three species gave a single fragment hybridized with 400 bp Pst I fragment of Gap b3 clone (Figure 7B). Therefore, Gap b3 gene is a single-copy gene, and mice and humans have genes homologous to the hamster-derived Gap b3.

The DNA molecules and antibodies in the present invention permit the detection of a tumor that results in the expression of Gap b3 at levels exceeding that expressed by non-transformed cells. Representative examples of cancers expressing elevated levels of Gap b3 include colorectal carcinoma, bladder carcinoma and certain hematopoietic cell malignancies. Within one embodiment, the method comprises measuring the level of RNA specific for Gap b3. RNA is isolated from a warm-blooded animal, such as a human, who may possess a tumor that results in the increased expression of Gap b3. The level of RNA specific for Gap b3 is measured in the isolated RNA sample. This level is compared with the level in control RNA from a warm-blooded animal. Suitable sources of control RNA, e.g., from non-transformed cells expressing Gap b3, are those whereby the expression of Gap b3 is not elevated. Measurement of the level of specific RNA in the sample from the warm-blooded animal may be prior to, simultaneous with, or subsequent to the measurement using the control RNA. A level of Gap b3 specific RNA in the sample which exceeds that in the control RNA indicates the presence of the tumor. It will be evident to one skilled in the art that a variety of methods exist for measuring a particular RNA.

A representative example of a suitable way to measure RNA specific for Gap b3 is by specific hybridization with a DNA fragment. Based upon the Gap b3 sequence information and material described above, nucleotide probes may be produced, e.g., by PCR amplification, and used for DNA or RNA diagnostic procedures (Landegren et al., Science 242:229, 1988) involving Gap b3. It will be evident to one skilled in the art that the probes may comprise a nucleotide sequence derived from DNA encoding the gene product, or a portion of such DNA. Oligodeoxynucleotides may be synthesized (Tan et al., Cold Spring Harbor Symp. Quant. Biol., Vol. 47, p. 383) or prepared with a DNA synthesizer, e.g., an Applied Biosystems DNA Synthesizer 380B.

For example, a DNA fragment (probe) capable of specifically hybridizing with RNA specific for Gap b3 is incubated with an RNA sample under conditions permitting hybridization. If hybridization has occurred, a pattern of hybridization is detected (e.g., by Northern blot analysis). The amount of hybridization is measured and compared to that for a sample of control RNA (as discussed above). An increased amount of hybridization, and, therefore, an increased level of RNA specific for Gap b3 is diagnostic for the presence of a tumor that results in the increased expression of Gap b3. The step of measuring the amount of hybridization may be performed by use of a reporter group bound to the probe, to a molecule that reacts with the probe, or to a second molecule that reacts with the first molecule. Suitable reporter groups include radioisotopes, fluorophores, enzymes, luminescers, and dye particles.

In another embodiment for the detection of a tumor that results in the elevated expression of Gap b3, antibodies to Gap b3 are employed. For example, a biological sample, such as tissue, taken from a warm-blooded animal is incubated with an antibody that specifically binds (i.e., which exhibits a binding affinity of about 10⁷ liters/mol) Gap b3, under conditions permitting immunocomplexes to form. Detection of immunocomplexes formed between Gap b3 and antibodies that specifically bind Gap b3 may be accomplished by a variety of known techniques, such as radioimmunoassays (RIA) and enzyme-linked immunosorbent assays (ELISA). The number of immunocomplexes formed using the biological sample is compared with that from a control sample in which the expression of Gap b3 is not elevated. A suitable control sample is from a warm-blooded animal with non-transformed cells expressing Gap b3. Measurement of the number of immunocomplexes in the sample may be prior to, simultaneous with, or subsequent to the measurement using the control sample. Numbers of immunocomplexes exceeding that for a control sample indicate the presence of the tumor.

Suitable immunoassays include the double monoclonal antibody sandwich immunoassay technique of David et al. (U.S. Patent 4,376,110); monoclonal-polyclonal antibody sandwich assays (Wide et al., in Kirkham and Hunter, eds., Radioimmunoassay Methods, E. and S. Livingstone, Edinburgh, 1970); the "western blot" method of Gordon et al. (U.S. Patent 4,452,901); immunoprecipitation of labeled ligand (Brown et al., J. Biol. Chem. 255:4980-4983, 1980); enzyme-linked immunosorbent assays as described by, for example, Raines and Ross (J. Biol. Chem. 257:5154-5160, 1982); immunocytochemical techniques, including the use of fluorochromes (Brooks et al., Clin. Exp. Immunol. 39: 477, 1980); and neutralization of activity [Bowen-Pope et al., Proc. Natl. Acad. Sci. USA 81:2396-2400 (1984)]. In addition to the immunoassays described above, a number of other immunoassays are available, including those described in U.S. Patent Nos.: 3,817,827; 3,850,752; 3,901,654; 3,935,074; 3,984,533; 3,996,345; 4,034,074; and 4,098,876.

For detection purposes, the antibodies may either be labeled or unlabeled. When unlabeled, the antibodies find use in agglutination assays. In addition, unlabeled antibodies can be used in combination with labeled molecules that are reactive with immunocomplexes, or in combination with labeled antibodies (second antibodies) that are reactive with the antibody directed against the Gap b3, such as antibodies specific for immunoglobulin. Alternatively, the antibodies can be directly labeled. Where they are labeled, the reporter group can include radioisotopes, fluorophores, enzymes, luminescers, or dye particles. These and other labels are well known in the art and are described, for example, in the following U.S. patents: 3,766,162; 3,791,932; 3,817,837; 3,996,345; and 4,233,402.

Typically in an ELISA assay, antigen is adsorbed to the surface of a microtiter well. Residual protein-binding sites on the surface are then blocked with an appropriate agent, such as bovine serum albumin (BSA), heat-inactivated normal goat serum (NGS), or BLOTTO (buffered solution of nonfat dry milk which also contains a preservative, salts, and an antifoaming agent). Typically, the well is then incubated with a sample suspected of containing specific antibody. However, an ELISA assay may also be used in the form of an inhibition assay to analyze a sample suspected of containing specific antigen, e.g., Gap b3. In an inhibition ELISA assay, a sample suspected of containing Gap b3 is applied, in combination with an antibody specific for Gap b3, to Gap b3 adsorbed to a solid support such as a microtiter well. The sample can be applied neat, or, more often, it can be diluted, usually in a buffered solution which contains a small amount (0.1%-5.0% by weight) of protein, such as BSA, NGS, or BLOTTO. After incubating for a sufficient length of time to allow specific binding to occur, the well is washed to remove "unbound" antibody (i.e., antibody not bound to the Gap b3 adsorbed to a solid support, and includes antibody bound to Gap b3 in the sample). The antibody bound via Gap b3 to the solid support may be detected in a variety of ways. For example, the well may be incubated with an anti-species specific immunoglobulin antibody labeled with a reporter group. The reporter group can be chosen from a variety of enzymes, including horseradish peroxidase, beta-galactosidase, alkaline phosphatase, and glucose oxidase. Sufficient time is allowed for specific binding to occur, then the well is again washed to remove unbound conjugate, and the substrate for the enzyme is added. Color is allowed to develop and the optical density of the contents of the well is determined visually or instrumentally. In this inhibition ELISA, maximal color development will occur in the absence of Gap b3 in the sample. The presence of Gap b3 in the sample will decrease the antibody which binds to the Gap b3 adsorbed to a solid support, and thus decrease the amount of color measured.

In one preferred embodiment of this aspect of the present invention, a reporter group is bound to the antibody. The step of detecting immunocomplexes involves removing substantially any unbound antibody and then detecting the presence or absence of the reporter group. Unbound antibody is antibody which has not bound to Gap b3.

In another preferred embodiment, a reporter group is bound to a second antibody capable of binding to the antibodies specific for Gap b3. The step of detecting immunocomplexes involves (a) removing substantially any unbound antibody (i.e., antibody not bound to Gap b3), (b) adding the second antibody, (c) removing substantially any unbound second antibody and then (d) detecting the presence or absence of the reporter group. Where the antibody specific for Gap b3 is derived from a mouse, the second antibody is an anti-murine antibody.

In a third preferred embodiment for detecting immunocomplexes, a reporter group is bound to a molecule capable of binding to the immunocomplexes. The step of detecting involves (a) adding the molecule, (b) removing substantially any unbound molecule, and then (c) detecting the presence or absence of the reporter group. An example of a molecule capable of binding to the immunocomplexes is protein A.

It will be evident to one skilled in the art that a variety of methods for detecting the immunocomplexes may be employed within the present invention. Reporter groups suitable for use in any of the methods include radioisotopes, fluorophores, enzymes, luminescers, and dye particles.

Another aspect of the present invention based upon the cloning and characterization of Gap b3 is the preparation of DNA constructs and recombinant plasmids. As noted above, the term "DNA constructs" as used herein comprises segments of DNA which are combined and juxtaposed in a manner which would not otherwise exist in nature. More specifically, DNA constructs may comprise a DNA sequence encoding Gap b3 in which there has been one or more deletions, substitutions, additions, and/or insertions, relative to "isolated" DNA sequences. A portion of the DNA sequence may be derived from a genomic or cDNA clone. The DNA described herein may include a suitable promoter.

Within a preferred embodiment of the present invention, recombinant plasmids capable of integration into a eukaryotic host cell comprise a promoter followed downstream by a DNA sequence encoding Gap b3, which in turn is followed downstream by a polyadenylation signal. The DNA sequence may be cDNA or genomic DNA. The plasmids may be used to stably transfect (transform) cells and thereby establish a host cell (Current Protocols in Molecular Biology, Vol. 1 & 2, Wiley Interscience). One embodiment of a method for producing Gap b3 comprises introducing into a eukaryotic host cell a DNA sequence encoding Gap b3. The host cells are grown in an appropriate medium and the protein product encoded by the DNA sequence produced by the host cell is isolated. Preferred host cells include mammalian cells. Particularly preferred host cells include COS-1 cells. Suitable methods for introducing cloned DNA sequences into cultured mammalian cells include calcium phosphate mediated transfection (e.g., Wigler et al., Cell 14:725, 1978; Corsaro and Pearson, Somatic Cell Genetics 7:603, 1981; Graham and Van der Eb, Virology 52:456, 1973). It will be evident to one skilled in the art that it is not necessary to use the entire sequence when producing recombinant Gap b3 proteins.

The following examples are offered by way of illustration and not by way of limitation.

### EXAMPLES

### EXAMPLE 1

### Purification of Galactoprotein b3

### A. Preparation of Galactoprotein b Fraction

Hamster embryo fibroblast cells transformed by polyoma virus ("NILpy cells") (20 ml packed) were homogenized in 100 ml (at 0°C) of 20 mM Tris-HCl buffer (pH 7.6) containing 0.34 M sucrose, 1 mM ATP, 1 mM EDTA, 0.2 mM PMSF and 0.2 mM leupeptin. The cell suspension was centrifuged at 25,000 rpm for 20 minutes. The pellet was extracted again with the same buffer and then solubilized with a detergent-containing buffer (50 mM Tris-HCl, pH 7.6, 25 mM KCl, 5 mM CaCl₂, 5 mM MgCl₂, 1% Empigen BB, 0.2 mM PMSF and 0.2 mM leupeptin). The mixture was incubated at 0°C for 1 hour, and insoluble materials were removed by centrifugation at 25,000 rpm for 40 minutes. The supernatant thus obtained was applied to a column (15 ml) of RCA (Ricinus communis lectin) conjugated to Sepharose®. The RCA-Sepharose was prepared by coupling RCA to a glutaraldehyde derivative of polyacrylhydrazido-Sepharose® (described in Wilchek and Miron, Meth. Enzymol. 34:72-76, 1974), followed by NaBH₄ reduction (described in Carter and Sharon, Arch. Biochem. Biophys. 180:570-582, 1977). After the supernatant was applied to the RCA-Sepharose column and the column washed extensively with phosphate buffered saline containing 0.5% Empigen BB, the bound materials were eluted with the same buffer containing 0.3 M lactose to yield the galactoprotein b fraction.

### B. Isolation of Galactoprotein b3 to Homogeneity

The galactoprotein b (Gap b) eluate from the RCA-Sepharose column was separated on preparative sodium dodecyl sulfate-polyacrylamide gel electrophoresis (SDS-PAGE). The SDS-PAGE was performed by the procedure according to Laemmli (Nature 227:680-685, 1970) using 2 mm slab gels. After electrophoresis, bands corresponding to Gap b were removed by cutting the gel, and glycoproteins were electrophoretically extracted (described in Stralfors and Belfrage, Anal. Biochem. 128:7-10, 1983), except that the buffer without agarose was used for electrophoresis. Bands corresponding to Gap b were identified by their increased expression at the surface of NILpy cells as detected by tritium-labeling of the surface galactoproteins. NILpy cells were labeled by the galactose oxidase/NaB³H₄ method (Gahmberg and Hakomori, J. Biol. Chem. 250:2447-2451, 1975). Tritium-labeled glycoproteins on gels were detected by fluorography using EN³HANCE (New England Nuclear, Boston, Mass.). Two-dimensional (2-D) SDS-PAGE under non-reducing (1st dimension) and reducing (2nd dimension) conditions was carried out according to the procedure of Kunicki et al. (J. Biol. Chem. 263:4516-4519, 1988).

### EXAMPLE 2

### Protein Sequencing

To obtain the internal amino acid (a.a.) sequence, Gap b3 heavy chain was digested with Achromobacter protease I (Masaki et al., Biochim. Biophys. Acta 660:44-50, 1981) after reduction of disulfide bonds with DTT followed by S-pyridyl-ethylation (Cavins and Friedman, Anal. Biochem. 35:489-493, 1970; Rüegg and Rudinger, Meth. Enzymol. 47:111-116 , 1977). Peptides were separated on an Aquapore RP300 column (Applied Biosystems, Foster City, Calif.) with High Resolution Liquid Chromatography Model 1090M (Hewlett Packard). The N-terminal a.a. sequence of Gap b3 heavy chain and the internal peptides were analyzed with an Applied Biosystems Protein Sequencer Model 477A with PTH Analyzer Model 120A.

### EXAMPLE 3

### Cloning and Characterization of DNA Complementary to Galactoprotein b3 mRNA

### A. Preparation of Synthetic Oligodeoxynucleotide Probes According to Partial Amino Acid Sequence Data

Based on amino acid sequences of a few peptides released on Achromobacter endolysyl peptidase treatment (described in Example 2), synthetic oligodeoxynucleotides were prepared with an Applied Biosystems DNA Synthesizer 380B (Foster City, California).

### B. RNA and DNA Preparation

Total RNA was prepared by the guanidine-thiocyanate method (e.g., Chirgwin et al., Biochemistry 24:5294-5299, 1979). In brief, cell pellets were homogenized in guanidine-thiocyanate solution and ethanol-precipitated twice. After resuspension in a saline/SDS mixture, RNA was extracted with phenol and Seavag's mixture (chloroform/isoamyl alcohol, 24:1), followed by ethanol precipitation. The poly A+ fraction was selected by oligo-dT cellulose column chromatography (Maniatis et al., Molecular Cloning: A Laboratory Manual, 1982, Cold Springs Harbor Laboratory, New York). Genomic DNA was purified by digesting the tissues with proteinase K in the presence of SDS and EDTA, followed by extraction with Seavag's mixture and ethanol precipitation (Id.).

### C. cDNA Libraries

All reagents and enzymes for cDNA synthesis were from the Promega cDNA synthesis kit and were used as per the manufacturer's instructions. cDNA was synthesized with NILpy poly A+ RNA (5 µg) by the method of Gubler and Hoffman (Gene 25:263-269, 1983) using a random hexamer, instead of oligo-dT, as a primer. The cDNA was ligated with a phosphorylated Eco RI linker, digested with Eco RI, and electrophoresed on 1% agarose gel. The cDNA was size-selected (>2 kb) and recovered from the gel using Geneclean (Bio 101, LaJolla, Calif.), then ligated to the dephosphorylated Eco RI arms of the λgt10 vector. The ligated DNA was packaged in vitro with a packaging extract (Gigapack, Stratagene, San Diego, Calif.).

### D. Screening of λgt10 Library

1. Probe Preparation. Synthetic oligodeoxynucleotides were labeled with γ-[³²-P]ATP by polynucleotide kinase.
2. Screening. Fifteen plaques out of 5 X 10⁵ primary recombinant phages giving positive signals after autoradiography were purified by successive plating and rescreening.
3. Additional Clones. To obtain additional clones covering the N-terminal region of Gap b3, a primer extension cDNA library was constructed using poly A⁺ RNA (15 µg) from NILpy cells and a 17-mer of oligodeoxynucleotide (5′ GCCCTCAAATGGAGCTC 3′, 0.7 µg) as a primer. The cDNA was ligated with λgt10 arms and packaged as described above. This cDNA library was screened with ³²P-labeled 170 bp Pst I fragment of clone #108 (5′ terminus of clone #108).

### E. Northern and Southern Hybridizations

For Northern blot analysis, 50 µg RNA or 5 µg poly A⁺ RNA was electrophoresed through a denaturing formaldehyde-agarose gel and transferred onto a Nylon membrane (Hybond-N, Amersham, Arlington Heights, Ill.).

For Southern blot analysis, 10 µg of genomic DNA was digested overnight with the appropriate restriction endonuclease (Eco RI, Hind III, or Bam HI) and loaded onto a 1% agarose gel. After electrophoresis, the gels were denatured (20 min.) in 0.5N NaOH and 1.5M NaCl, neutralized (30 min., 2x) in 0.5M Tris-HCl (pH 7.5) 1.5M NaCl. The DNA was successively transferred onto a Nylon membrane by capillary action and fixed by UV irradiation (Maniatis et al., Molecular Cloning: A Laboratory Manual, 1982, Cold Springs Harbor Laboratory, New York).

Both Northern and Southern membrane filters were prehybridized in 50% formamide, 5x SSPE, 5x Denhardt's solution, and 0.1% SDS solution at 42°C for two hours and then hybridized overnight at 42°C with a ³²P-random primed-labeled (Feinberg and Vogelstein, Anal. Biochem. 132:6, 1983; Anal. Biochem. 137:266, 1984) 400 bp DNA fragment obtained by digestion of clone #104 with Pst 1. Membrane filters were washed in 2x SSC, 0.1% SDS at room temperature 5 min. twice and then in 1x SSC, 0.05% SDS at 68°C for one hour.

### F. Subcloning and Restriction Enzyme Mapping

DNA from the phage clones was digested with Eco RI and ligated with dephosphorylated Eco RI arms of pT7T3 18U plasmid (Pharmacia LKB Biotechnology, Inc.). After DNA transformation of XL-1 Blue strain bacteria (Stratagene), the clones with insert were screened by color selection with isopropyl-1-thio-β-D-galactopyranoside (IPTG) and X-Gal. Restriction enzymes were obtained from BRL or New England Biolabs.

### G. DNA Sequencing

Dideoxynucleotide termination sequencing reactions (Sanger et al., Proc. Natl. Acad. Sci. USA 74:5463-5467, 1977) were performed with single-strand DNA of Phagescript clones or pT7T3 clones obtained by super-infection with a helper phage, M13K07 (Vieira and Messing, Meth. Enzymol. 153:3-34, 1987). The sequencing strategy is shown in Figure 3. DNA sequencing was carried out with a Model 373A DNA sequencer (Applied Biosystems, Foster City, California) using Sequenase (United States Biochemical Corp., Cleveland, Ohio) and an M13 universal primer or synthetic oligodeoxynucleotide primers. IBI Pustell Sequence Analysis Software (MS-DOS version) was used for sequence analysis.

### H. Cloning and Characterization of DNA Complementary to mRNA of Gap b3 from Human Cells

WI38 (human foreskin fibroblast, ATCC CCL75), WI38/VA13 (SV-40 transformant of WI38, ATCC CCL 75.1), BALB/3T3 (murine embryonal fibroblast, ATCC CCL 163), SV-T2 (SV-40 transformant of BALB/3T3, ATCC CCL 163.1), EJ-1 (human bladder carcinoma, JCRB 0710), and T24 (human bladder carcinoma, JCRB 0711) were supplied by the Japanese Cancer Research Resources Bank (JCRB, Tokyo, Japan). All cell lines were cultured in Dulbecco's modified MEM (D-MEM) supplemented with 10% fetal bovine serum at 37°C under 5% CO₂.

Total RNA was prepared according to Section B above. The poly A⁺ fraction was also selected according to Section B above, using two cycles of oligo (dT)-cellulose column chromatography. The construction of cDNA libraries was performed as described in Section C above, except that cDNA was synthesized with T24 poly A⁺ RNA, using a Pharmacia (Uppsala, Sweden) cDNA synthesis kit.

Primary recombinant phages (2 x 10⁵) were screened by hybridization with ³²P-random prime-labeled (Feinberg and Vogelstein, Anal. Biochem. 132:6-13, 1983) 400 bp DNA fragment obtained by digestion of hamster Gap b3 clone 104 with Pst I. Twenty plaques giving positive signals after autoradiography were purified by successive plating and rescreening. To obtain additional clones covering the N-terminal region of human Gap b3, the same library was screened with ³²P-labeled 180 bp Pst I fragment of human Gap b3 clone 1 (5′ terminus of clone 1).

Northern hybridizations were performed as described in Section E above, except that 4 µg of poly A⁺ RNA was used.

Subcloning was performed according to Section F above. DNA sequencing was performed as described in Section G above. A set of deletions was prepared according to the methodology of Henikoff (Gene [Amst.] 28:351-359, 1984) and by use of restriction sites. The sequencing strategy is shown in Figure 5.

### EXAMPLE 4

### Preparation and Characterization of MAbs Directed to Galactoprotein b3

### A. Generation of NAbs

Production of MAbs directed to galactoprotein b3 are obtained by immunization of 3-month-old BALB/c mice. Mice are immunized with galactoprotein b3 (prepared as described in Example 1) emulsified in Ribi's adjuvant (monophosphoryl lipid A + trehalose dimycolate) by intraperitoneal injection 4 times (3 week interval), with approximately µg of Gap b3 per injection. Spleen cells are fused with NS-1 myeloma cells 3 days after the last immunization, and hybridomas are cloned by limiting dilution at least 3 times. Hybridomas are screened by particle-concentrated fluorescence immunoassay (PCFI). Isotope and subclass are determined by PCFI using goat anti-mouse fluorescein isothiocyanate (FITC) - conjugated antibodies, as well as by the Ochterlony method of using rabbit anti-mouse antibodies (Boehringer Mannheim Biochemicals). Antibodies are purified on a protein A Sepharose 4B column (pH 9.0) eluted with 100 mM citrate buffer (pH 4.2), and dialyzed against 20 mM Tris buffer (pH 7.4).

### B. PCFI Screening

Approximately 50 µg of purified Gap b3 (prepared as described in Example 1) is mixed with 1 ml of 0.5% (w/v) Fluoricon Carboxyl-Polystyrene Assay Particles (0.86 µm, Pandex) and covalently coupled by adding solid 1-ethyl-3[3-dimethyl-aminopropyl] carbodiimide to give a final concentration of 1 mg/ml. After vortexing, the mixture is incubated at room temperature for 1-2 hours. The microparticles are then centrifuged (3,000xg, 10 min.), washed with phosphate-buffered saline (PBS), blocked with either bovine serum albumin (BSA)/PBS 5% or human serum (1:10 dilution), and brought to final volume of 0.25% w/v in PBS. Antigen-coated particles are then diluted 1:10 in BSA-coated particles (similar procedure) to give a final particle concentration of 0.225% BSA particles and 0.025% Gap b3 particles. Twenty µl of BSA-Gap b3 or BSA-coated particles are distributed in 96-well Epicon assay plates (Pandex) with a 0.2 µm filter. The automated particle concentrated fluorescence immunoassay screen machine (Pandex) (as described in Jolley et al., J. Immunol. Meth. 67:21-35,1984) performs the following steps sequentially by vacuum suction through the 0.2 µm filter in the bottom of each well and distribution of buffers through an 9-channel pump: incubation for 10 min. with 50 µl of Mab culture supernatant, washing with PBS, incubation for 10 min. with 25 µl of affinity-purified goat anti-mouse Ig FITC-conjugated antibody (1:200, Pandex), washing with PBS, and reading at 485 mm/535 nm after final suction centering and concentrating antigencoated particles in the bottom of wells.

From the foregoing, it will be evident that, although specific embodiments of the invention have been described herein for purposes of illustration, various modifications may be made without deviating from the scope of the invention.

## Claims

1. An isolated DNA molecule encoding galactoprotein b3 said protein obtainable from hamster fibroblast NIL cells transformed with polyoma virus and said protein having an approximate molecular weight of 140,000 as determined by sodium dodecyl sulfate-polyacrylamide gel electrophoresis under non-reducing conditions.

2. The isolated DNA molecule of Claim 1 wherein said DNA molecule encodes human galactoprotein b3.

3. A cDNA molecule according to Claim 1.

4. A genomic DNA molecule according to Claim 1.

5. A DNA molecule according to Claim 1 wherein said galactoprotein b3 comprises the amino acid sequence of Figure 6 from phenylalanine, amino acid 1, to tyrosine, amino acid 1019.

6. A DNA molecule according to Claim 1 wherein said galactoprotein b3 comprises a sequence of nucleotides as shown in Figure 6 from nucleotide 1 to nucleotide 3057.

7. An isolated DNA molecule capable of specifically hybridizing with a DNA molecule encoding galactoprotein b3, said protein obtainable from hamster fibroblast NIL cells transformed with polyoma virus and said protein having an approximate molecular weight of 140,000 as determined by sodium dodecyl sulfate-polyacrylamide gel electrophoresis under non-reducing conditions.

8. A DNA construct comprising a DNA sequence encoding galactoprotein b3, said protein obtainable from hamster fibroblast NIL cells transformed with polyoma virus and said protein having an approximate molecular weight of 140,000 as determined by sodium dodecyl sulfatepolyacrylamide gel electrophoresis under non-reducing conditions.

9. The DNA construct of Claim 8 wherein at least a portion of the DNA sequence is derived from a cDNA clone of galactoprotein b3.

10. The DNA construct of Claim 8 wherein at least a portion of the DNA sequence is derived from a genomic clone of galactoprotein b3.

11. A recombinant plasmid capable of expression in a host cell, said recombinant plasmid further comprising a DNA sequence encoding galactoprotein b3, said protein obtainable from hamster fibroblast NIL cells transformed with polyoma virus and said protein having an approximate molecular weight of 140,000 as determined by sodium dodecyl sulfate-polyacrylamide gel electrophoresis under non-reducing conditions.

12. The recombinant plasmid of Claim 11 wherein the DNA sequence comprises galactoprotein b3 cDNA.

13. The recombinant plasmid of Claim 11 wherein the DNA sequence comprises galactoprotein b3 genomic DNA.

14. A recombinant plasmid capable of integration in host cell DNA, said plasmid comprising a promoter followed downstream by a DNA sequence encoding galactoprotein b3, said protein obtainable from hamster fibroblast NIL cells transformed with polyoma virus and said protein having an approximate molecular weight of 140,000 as determined by sodium dodecyl sulfate-polyacrylamide gel electrophoresis under non-reducing conditions, said DNA sequence being followed downstream by a polyadenylation signal.

15. Eukaryotic cells stably transfected with a recombinant plasmid comprising a DNA sequence encoding galactoprotein b3, said protein obtainable from hamster fibroblast NIL cells transformed with polyoma virus and said protein having an approximate molecular weight of 140,000 as determined by sodium dodecyl sulfatepolyacrylamide gel electrophoresis under non-reducing conditions, said cells producing said galactoprotein b3 in recoverable amounts.

16. A method for producing galactoprotein b3, said protein obtainable from hamster fibroblast NIL cells transformed with polyoma virus and said protein having an approximate molecular weight of 140,000 as determined by sodium dodecyl sulfate-polyacrylamide gel electrophoresis under non-reducing conditions, comprising:
introducing into a eukaryotic host cell a DNA sequence encoding galactoprotein b3;
growing said host cell in an appropriate medium; and isolating the protein product, encoded by said DNA sequence, produced by said host cell.

17. The method of Claim 16 wherein the host cell is a mammalian cell.

18. The method of Claim 17 wherein the mammalian cell is COS-1.

19. An antibody that specifically binds to galactoprotein b3, said protein obtainable from hamster fibroblast NIL cells transformed with polyoma virus and said protein having an approximate molecular weight of 140,000 as determined by sodium dodecyl sulfatepolyacrylamide gel electrophoresis under non-reducing conditions.

20. The antibody of Claim 19 wherein the antibody is a monoclonal antibody.

21. A monoclonal antibody produced by a hybridoma formed by the fusion of cells from a myeloma line and spleen cells from an animal previously immunized with a substantially pure galactoprotein b3, said protein obtainable from hamster fibroblast NIL cells transformed with polyoma virus and said protein having an approximate molecular weight of 140,000 as determined by sodium dodecyl sulfate-polyacrylamide gel electrophoresis under non-reducing conditions.

22. A monoclonal antibody that competitively inhibits the formation of an immunocomplex between the antibody of Claim 19 and galactoprotein b3.

23. A method for the detection of a tumor that results in the expression of galactoprotein b3, said protein obtainable from hamster fibroblast NIL cells transformed with polyoma virus and said protein having an approximate molecular weight of 140,000 as determined by sodium dodecyl sulfate-polyacrylamide gel electrophoresis under non-reducing conditions, at levels exceeding that expressed by non-transformed cells, comprising the steps of:
(a) measuring the level of RNA specific for galactoprotein b3 in RNA isolated from a warm-blooded animal;
(b) measuring the level of RNA specific for galactoprotein b3 in control RNA isolated from nontransformed, galactoprotein b3-expressing cells from a warm-blooded animal; and
(c) comparing the levels of RNA measured in steps (a) and (b), and thereby determining the presence or absence of the tumor.

24. The method of Claim 23 wherein step (a) of measuring the level of RNA specific for galactoprotein b3 comprises:
(a) incubating said RNA with a DNA fragment capable of specifically hybridizing with RNA specific for galactoprotein b3 under conditions and for a time sufficient to allow hybridization; and
(b) measuring the amount of said hybridization.

25. The method of Claim 23 wherein step (b) of measuring the level of RNA specific for galactoprotein in control RNA comprises:
(a) incubating said control RNA with a DNA fragment capable of specifically hybridizing with RNA specific for galactoprotein b3 under conditions and for a time sufficient to allow hybridization; and
(b) measuring the amount of said hybridization.

26. The method of Claim 24 or 25 wherein a reporter group is bound to said DNA fragment.

27. The method of Claim 26 wherein said reporter group is a radioisotope.

28. A method for the detection of a tumor that results in the expression of galactoprotein b3, said protein obtainable from hamster fibroblast NIL cells transformed with polyoma virus and said protein having an approximate molecular weight of 140,000 as determined by sodium dodecyl sulfate-polyacrylamide gel electrophoresis under non-reducing conditions, at levels exceeding that expressed by non-transformed cells, comprising the steps of:
(a) incubating a biological sample from a warm-blooded animal, said sample suspected of containing galactoprotein b3, with an antibody that specifically binds to galactoprotein b3 under conditions and for a time sufficient to allow immunocomplexes to form therebetween;
(b) detecting immunocomplexes formed between said antibody and galactoprotein b3 in said biological sample;
(c) incubating a control biological sample, wherein said control sample is from a warm-blooded animal possessing non-transformed cells expressing galactoprotein b3 with an antibody that specifically binds to galactoprotein b3 under conditions and for a time sufficient to allow immunocomplexes to form therebetween;
(d) detecting immunocomplexes formed between said antibody and galactoprotein b3 in said control biological sample; and
(e) comparing the number of immunocomplexes detected in steps (b) and (d), and thereby determining the presence or absence of the tumor.

29. The method of claim 28 wherein a reporter group is bound to a second antibody capable of binding to the antibody, and wherein the step of detecting comprises
(a) removing substantially any unbound antibody,
(b) incubating with the second antibody under conditions and for a time sufficient to allow immunocomplexes to form,
(c) removing substantially any unbound second antibody, and
(d) detecting the presence or absence of the reporter group.

30. The method of claim 28 wherein a reporter group is bound to a molecule capable of binding to the immunocomplexes, and wherein the step of detecting comprises
(a) incubating with the molecule under conditions and for a time sufficient to allow binding to the immunocomplexes,
(b) removing substantially any unbound molecule, and
(c) detecting the presence or absence of the reporter group.

31. The method of claim 28 wherein a reporter group is bound to the antibody, and wherein the step of detecting comprises removing substantially any unbound antibody and thereafter detecting the presence or absence of the reporter group.

32. The method of claim 29, 30, or 31 wherein the reporter group is selected from the group consisting of radioisotopes, fluorophores, enzymes, luminescers, and dye particles.

## Patentansprüche

1. Ein isoliertes DNA-Molekül, das Galactoprotein b3 kodiert, wobei besagtes Protein aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen.

2. Das isolierte DNA-Molekül von Anspruch 1, wobei besagtes DNA-Molekül menschliches Galactoprotein b3 kodiert.

3. Ein cDNA-Molekül nach Anspruch 1.

4. Ein Genom-DNA-Molekül nach Anspruch 1.

5. Ein DNA-Molekül nach Anspruch 1, wobei besagtes Galactoprotein b3 die Aminosäuresequenz von Figur 6 von Phenylalanin, Aminosäure 1, bis Tyrosin, Aminosäure 1019, umfaßt.

6. Ein DNA-Molekül nach Anspruch 1, wobei besagtes Galactoprotein b3 eine Sequenz von Nukleotiden umfaßt, wie dargestellt in Figur 6, von Nukleotid 1 bis Nukleotid 3057.

7. Ein isoliertes DNA-Molekül, das in der Lage ist, spezifisch mit einem DNA-Molekül zu hybridisieren, das Galactoprotein b3 kodiert, wobei besagtes Protein aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen.

8. Ein DNA-Konstrukt, das eine DNA-Sequenz umfaßt, die Galactoprotein b3 kodiert, wobei besagtes Protein erhältlich ist aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen.

9. Das DNA-Konstrukt von Anspruch 8, wobei wenigstens ein Abschnitt der DNA-Sequenz abgeleitet ist von einem cDNA-Klon von Galactoprotein b3.

10. Das DNA-Konstrukt von Anspruch 8, wobei wenigstens ein Abschnitt der DNA-Sequenz abgeleitet ist von einem Genom-Klon von Galactoprotein b3.

11. Ein rekombinantes Plasmid, das zur Expression in einer Wirtszelle in der Lage ist, wobei besagtes rekombinantes Plasmid außerdem eine DNA-Sequenz umfaßt, die Galactoprotein b3 kodiert, wobei besagtes Protein aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen.

12. Das rekombinante Plasmid von Anspruch 11, wobei die DNA-Sequenz Galactoprotein b3-cDNA umfaßt.

13. Das rekombinante Plasmid von Anspruch 11, wobei die DNA-Sequenz Galactoprotein b3-Genom-DNA umfaßt.

14. Ein rekombinantes Plasmid, das zur Integration in Wirtszell-DNA in der Lage ist, wobei besagtes Plasmid einen Promotor umfaßt, flußabwärts gefolgt von einer DNA-Sequenz, die Galactoprotein b3 kodiert, wobei besagtes Protein aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen, wobei besagter DNA-Sequenz flußabwärts ein Polyadenylierungssignal folgt.

15. Eukaryontische Zellen, die mit einem rekombinanten Plasmid stabil transfiziert sind, das eine DNA-Sequenz umfaßt, die Galactoprotein b3 kodiert, wobei besagtes Protein erhältlich aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen, wobei besagte Zellen besagtes Galactoprotein b3 in gewinnbaren Mengen produzieren.

16. Ein Verfahren zur Herstellung von Galactoprotein b3, wobei besagtes Protein aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen, welches umfaßt:
Einführen einer DNA-Sequenz, die Galactoprotein b3 kodiert, in eine eukaryontische Wirtszelle;
Züchten besagter Wirtszelle in einem geeigneten Medium; und Isolieren des von besagter DNA-Sequenz kodierten Proteinproduktes, das von besagter Wirtszelle produziert ist.

17. Das Verfahren von Anspruch 16, wobei die Wirtszelle eine Säugetierzelle ist.

18. Das Verfahren von Anspruch 17, wobei die Säugetierzelle COS-1 ist.

19. Ein Antikörper, der sich spezifisch an Galactoprotein b3 bindet, wobei besagtes Protein aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen.

20. Der Antikörper von Anspruch 19, wobei der Antikörper ein monoklonaler Antikörper ist.

21. Ein monoklonaler Antikörper, der von einem Hybridom produziert ist, das gebildet ist durch die Fusion von Zellen aus einer Myelomlinie und Milzzellen von einem Tier, das zuvor mit einem im wesentlichen reinen Galactoprotein b3 immunisiert worden ist, wobei besagtes Protein aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen.

22. Ein monoklonaler Antikörper, der kompetitiv die Bildung eines Immunkomplexes zwischen dem Antikörper von Anspruch 19 und Galactoprotein b3 inhibiert.

23. Ein Verfahren zum Nachweis eines Tumors, der zur Expression von Galactoprotein b3 führt, wobei besagtes Protein aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen, in Gehalten, die diejenigen übersteigen, die von nichttransformierten Zellen exprimiert werden, welches die Schritte umfaßt:
(a) Messen des Gehaltes von RNA, die für Galactoprotein b3 spezifisch ist, in aus einem Warmblüter isolierter RNA;
(b) Messen des Gehaltes von RNA, die für Galactoprotein b3 spezifisch ist, in Kontroll-RNA, die aus nichttransformierten, Galactoprotein b3-exprimierenden Zellen von einem Warmblüter isoliert ist; und
(c) Vergleichen der in den Schritten (a) und (b) gemessenen Gehalte von RNA und dadurch Bestimmen des Vorhandenseins und des Nicht-Vorhandenseins des Tumors.

24. Das Verfahren von Anspruch 23, wobei Schritt (a) des Messens des Gehaltes von RNA, die für Galactoprotein b3 spezifisch ist, umfaßt:
(a) Inkubieren besagter RNA mit einem DNA-Fragment, das in der Lage ist, spezifisch mit RNA zu hybridisieren, die für Galactoprotein b3 spezifisch ist, unter Bedingungen und für einen Zeitraum, die ausreichend sind, um Hybridisierung zu ermöglichen; und
(b) Messen der Menge besagter Hybridisierung.

25. Das Verfahren von Anspruch 23, wobei Schritt (b) des Messens des Gehaltes von RNA, die für Galactoprotein spezifisch ist, in Kontroll-RNA umfaßt:
(a) Inkubieren besagter Kontroll-RNA mit einem DNA-Fragment, das in der Lage ist, spezifisch mit RNA zu hybridisieren, die für Galactoprotein b3 spezifisch ist, unter Bedingungen und für einen Zeitraum, die ausreichend sind, um Hybridisierung zu ermöglichen; und
(b) Messen der Menge besagter Hybridisierung.

26. Das Verfahren von Anspruch 24 oder 25, wobei eine Reporter-Gruppe an besagtes DNA-Fragment gebunden ist.

27. Das Verfahren von Anspruch 26, wobei besagte Reporter-Gruppe ein Radioisotop ist.

28. Ein Verfahren zum Nachweis eines Tumors, der zur Expression von Galactoprotein b3 führt, wobei besagtes Protein aus Hamster-Fibroblasten-NIL-Zellen gewonnen werden kann, die mit Polyoma-Virus transformiert sind, und besagtes Protein eine ungefähre relative Molekülmasse von 140.000 besitzt, wie bestimmt mit Natriumdodecylsulfat-Polyacrylamid-Gelelektrophorese unter nicht-reduzierenden Bedingungen, in Gehalten, die diejenigen übersteigen, die von nichttransformierten Zellen exprimiert werden, welches die Schritte umfaßt:
(a) Inkubieren einer biologischen Probe von einem Warmblüter, wobei bei besagter Probe der Verdacht besteht, daß sie Galactoprotein b3 enthält, mit einem Antikörper, der sich spezifisch an Galactoprotein b3 bindet, unter Bedingungen und für einen Zeitraum, die ausreichend sind, um zu ermöglichen, daß sich zwischen dieser Immunkomplexe bilden;
(b) Nachweisen von zwischen besagtem Antikörper und Galactoprotein b3 in besagter biologischer Probe gebildeten Immunkomplexen;
(c) Inkubieren einer biologischen Kontrollprobe, wobei besagte Kontrollprobe von einem Warmblüter stammt, der nicht-transformierte Zellen besitzt, die Galactoprotein b3 exprimieren, mit einem Antikörper, der sich spezifisch an Galactoprotein b3 bindet, unter Bedingungen und für einen Zeitraum, die ausreichend sind, um zu ermöglichen, daß sich zwischen diesen Immunkomplexe bilden;
(d) Nachweisen von zwischen besagtem Antikörper und Galactoprotein b3 in besagter biologischen Kontrollprobe gebildeten Immunkomplexen; und
(e) Vergleichen der Zahl von Immunkomplexen, die in den Schritten (b) und (d) nachgewiesen sind, und dadurch Bestimmen des Vorhandenseins oder Nicht-Vorhandenseins des Tumors.

29. Das Verfahren von Anspruch 28, wobei eine Reporter-Gruppe an einen zweiten Antikörper gebunden ist, der in der Lage ist, sich an den Antikörper zu binden, und wobei der Nachweis-Schritt (a) Entfernen von im wesentlichen jeglichem nichtgebundenen Antikörper, (b) Inkubieren mit dem zweiten Antikörper unter Bedingungen und für einen Zeitraum, die ausreichend sind, um es zu ermöglichen, daß sich Immunkomplexe bilden, (c) Entfernen von im wesentlichen jeglichem nichtgebundenen zweiten Antikörper und (d) Nachweisen des Vorhandenseins oder Nicht-Vorhandenseins der Reporter-Gruppe umfaßt.

30. Das Verfahren von Anspruch 28, wobei eine Reporter-Gruppe an ein Molekül gebunden ist, das in der Lage ist, sich an die Immunkomplexe zu binden, und wobei der Nachweis-Schritt (a) Inkubieren mit dem Molekül unter Bedingungen und für einen Zeitraum, die ausreichend sind, um Bindung an die Immunkomplexe zu ermöglichen, (b) Entfernen von im wesentlichen jeglichem nicht-gebundenen Molekül und (c) Nachweisen des Vorhandenseins oder Nicht-Vorhandenseins der Reporter-Gruppe umfaßt.

31. Das Verfahren von Anspruch 28, wobei eine Reporter-Gruppe an den Antikörper gebunden ist und wobei der Nachweis-Schritt Entfernen von im wesentlichen jeglichem nicht-gebundenen Antikörper und danach Nachweisen des Vorhandenseins oder Nicht-Vorhandenseins der Reporter-Gruppe umfaßt.

32. Das Verfahren von Anspruch 29, 30 oder 31, wobei die Reporter-Gruppe ausgewählt ist aus der Gruppe, die aus Radioisotopen, Fluorophoren, Enzymen, Lumineszeren und Farbstoffteilchen besteht.

## Revendications

1. Molécule d'ADN isolée codant pour la galactoprotéine b3, ladite protéine pouvant être obtenue à partir de cellules NIL dérivées de fibroblastes de hamster, transformées avec un virus de polyome, ladite protéine ayant un poids moléculaire approximatif de 140 000 déterminé par électrophorèse sur gel de polyacrylamide au dodécylsulfate de sodium dans des conditions non réductrices.

2. Molécule d'ADN isolée suivant la revendication 1, qui code pour la galactoprotéine b3 humaine.

3. Molécule d'ADNc suivant la revendication 1.

4. Molécule d'ADN génomique suivant la revendication 1.

5. Molécule d'ADN suivant la revendication 1, la galactoprotéine b3 comprenant la séquence d'amino-acides de la Figure 6 allant de l'amino-acide 1 consistant en phénylalanine à l'amino-acide 1019 consistant en tyrosine.

6. Molécule d'ADN suivant la revendication 1, la galactoprotéine b3 comprenant une séquence de nucléotides telle que représentée sur la Figure 6 allant du nucléotide 1 jusqu'au nucléotide 3057.

7. Molécule d'ADN isolée apte à l'hybridation spécifique avec une molécule d'ADN codant pour la galactoprotéine b3, ladite protéine pouvant être obtenue à partir de cellules NIL dérivées de fibroblastes de hamster, transformées avec un virus de polyome, ladite protéine ayant un poids moléculaire approximatif de 140 000 déterminé par électrophorèse sur gel de polyacrylamide au dodécylsulfate de sodium dans des conditions non réductrices.

8. Produit d'assemblage d'ADN comprenant une séquence d'ADN codant pour la galactoprotéine b3, ladite protéine pouvant être obtenue à partir de cellules NIL dérivées de fibroblastes de hamster, transformées avec un virus de polyome, ladite protéine ayant un poids moléculaire approximatif de 140 000 déterminé par électrophorèse sur gel de polyacrylamide au dodécylsulfate de sodium dans des conditions non réductrices.

9. Produit d'assemblage d'ADN suivant la revendication 8, dans lequel au moins une portion de la séquence d'ADN est dérivée d'un clone d'ADNc de galactoprotéine b3.

10. Produit d'assemblage d'ADN suivant la revendication 8, dans lequel au moins une portion de la séquence d'ADN est dérivée d'un clone génomique de galactoprotéine b3.

11. Plasmide recombinant apte à l'expression dans une cellule-hôte, ledit plasmide recombinant comprenant en outre une séquence d'ADN codant pour la galactoprotéine b3, ladite protéine pouvant être obtenue à partir de cellules NIL dérivées de fibroblastes de hamster, transformées avec un virus de polyome, et ladite protéine ayant un poids moléculaire approximatif de 140 000 déterminé par électrophorèse sur gel de polyacrylamide au dodécylsulfate de sodium dans des conditions non réductrices.

12. Plasmide recombinant suivant la revendication 11, dans lequel la séquence d'ADN comprend l'ADNc de galactoprotéine b3.

13. Plasmide recombinant suivant la revendication 11, dans lequel la séquence d'ADN comprend l'ADN génomique de galactoprotéine b3.

14. Plasmide recombinant apte à l'intégration dans l'ADN d'une cellule-hôte, ledit plasmide comprenant un promoteur suivi en aval par une séquence d'ADN codant pour la galactoprotéine b3, ladite protéine pouvant être obtenue à partir de cellules NIL dérivées de fibroblastes de hamster, transformées avec un virus de polyome, et ladite protéine possédant un poids moléculaire approximatif de 140 000 déterminé par électrophorèse sur gel de polyacrylamide au dodécylsulfate de sodium dans des conditions non réductrices, ladite séquence d'ADN étant suivie en aval par un signal de polyadénylation.

15. Cellules eucaryotiques transfectées de manière stable avec un plasmide recombinant comprenant une séquence d'ADN codant pour la galactoprotéine b3, ladite protéine pouvant être obtenue à partir de cellules NIL dérivées de fibroblastes de hamster, transformées avec un virus de polyome, et ladite protéine ayant un poids moléculaire approximatif de 140 000 déterminé par électrophorèse sur gel de polyacrylamide au dodécylsulfate de sodium dans des conditions non réductrices, lesdites cellules produisant ladite galactoprotéine b3 en des quantités aptes à la séparation.

16. Procédé de production de galactoprotéine b3, ladite protéine pouvant être obtenue à partir de cellules NIL dérivées de fibroblastes de hamster, transformées avec un virus de polyome, et ladite protéine ayant un poids moléculaire approximatif de 140 000 déterminé par électrophorèse sur gel de polyacrylamide au dodécylsulfate de sodium dans des conditions non réductrices, comprenant :
l'introduction dans une cellule-hôte eucaryotique d'une séquence d'ADN codant pour la galactoprotéine b3 ;
la culture de ladite cellule-hôte dans un milieu approprié ; et l'isolement du produit protéique, codé par ladite séquence d'ADN, engendré par ladite cellule-hôte.

17. Procédé suivant la revendication 16, dans lequel la cellule-hôte est une cellule de mammifère.

18. Procédé suivant la revendication 17, dans lequel la cellule de mammifère est une cellule COS-1.

19. Anticorps qui se lie spécifiquement à la galactoprotéine b3, ladite protéine pouvant être obtenue à partir de cellules NIL dérivées de fibroblastes de hamster, transformées avec un virus de polyome, et ladite protéine ayant un poids moléculaire approximatif de 140 000 déterminé par électrophorèse sur gel de polyacrylamide au dodécylsulfate de sodium dans des conditions non réductrices.

20. Anticorps suivant la revendication 19, qui est un anticorps monoclonal.

21. Anticorps monoclonal produit par un hybridome formé par fusion de cellules provenant d'une lignée de myélome et de cellules spléniques provenant d'un animal immunisé préalablement avec une galactoprotéine b3 pratiquement pure, ladite protéine pouvant être obtenue à partir de cellules NIL dérivées de fibroblastes de hamster, transformées avec un virus de polyome, et ladite protéine ayant un poids moléculaire approximatif de 140 000 déterminé par électrophorèse sur gel de polyacrylamide au dodécylsulfate de sodium dans des conditions non réductrices.

22. Anticorps monoclonal qui inhibe de manière compétitive la formation d'un immun-complexe entre l'anticorps suivant la revendication 19 et la galactoprotéine b3.

23. Procédé de détection d'une tumeur ayant pour résultat l'expression de la galactoprotéine b3, ladite protéine pouvant être obtenue à partir de cellules NIL dérivées de fibroblastes de hamster, transformées avec le virus de polyome, et ladite protéine ayant un poids moléculaire approximatif de 140 000 déterminé par électrophorèse sur gel de polyacrylamide au dodécylsulfate de sodium dans des conditions non réductrices, à des teneurs excédant celle exprimée par les cellules non transformées, comprenant les étapes :
(a) de mesure de la teneur en ARN spécifique pour la galactoprotéine b3 dans l'ARN isolé d'un animal à sang chaud ;
(b) de mesure de la teneur en ARN spécifique pour la galactoprotéine b3 dans un ARN témoin isolé de cellules non transformées exprimant la galactoprotéine b3, provenant d'un animal à sang chaud ; et
(c) de comparaison des teneurs d'ARN mesurées dans les étapes (a) et (b) et, ainsi, de détermination de la présence ou de l'absence de la tumeur.

24. Procédé suivant la revendication 23, dans lequel l'étape (a) de mesure de la teneur en ARN spécifique de la galactoprotéine b3 comprend :
(a) la mise en incubation dudit ARN avec un fragment d'ADN capable de s'hybrider spécifiquement avec l'ARN spécifique pour la galactoprotéine b3 dans des conditions et pendant un temps suffisants pour permettre l'hybridation ; et
(b) la mesure du taux de ladite hybridation.

25. Procédé suivant la revendication 23, dans lequel l'étape (b) de mesure de la teneur en ARN spécifique de la galactoprotéine dans l'ARN témoin comprend :
(a) la mise en incubation dudit ARN témoin avec un fragment d'ADN capable de s'hybrider spécifiquement avec l'ARN spécifique de la galactoprotéine b3 dans des conditions et pendant un temps suffisants pour permettre l'hybridation ; et
(b) la mesure du taux de ladite hybridation.

26. Procédé suivant la revendication 24 ou 25, dans lequel un groupe rapporteur est lié au fragment d'ADN.

27. Procédé suivant la revendication 26, dans lequel le groupe rapporteur est un radio-isotope.

28. Procédé de détection d'un tumeur qui a pour résultat l'expression de la galactoprotéine b3, ladite protéine pouvant être obtenue à partir de cellules NIL dérivées de fibroblastes de hamster, transformées avec le virus de polyome, et ladite protéine ayant un poids moléculaire approximatif de 140 000 déterminé par électrophorèse sur gel de polyacrylamide au dodécylsulfate de sodium dans des conditions non réductrices, à des teneurs excédant celle exprimée par des cellules non transformées, comprenant les étapes :
(a) de mise en incubation d'un échantillon biologique provenant d'un animal à sang chaud, ledit échantillon étant suspecté de contenir de la galactoprotéine b3, avec un anticorps qui se lie spécifiquement à la galactoprotéine b3 dans des conditions et pendant un temps suffisants pour permettre la formation d'immun-complexes entre la galactoprotéine et l'anticorps ;
(b) de détection des immun-complexes formés entre ledit anticorps et la galactoprotéine b3 dans ledit échantillon biologique ;
(c) de mise en incubation d'un échantillon biologique témoin, ledit échantillon témoin provenant d'un animal à sang chaud possédant des cellules non transformées exprimant la galactoprotéine b3, avec un anticorps qui se lie spécifiquement à la galactoprotéine b3 dans des conditions et pendant un temps suffisants pour permettre la formation d'immun-complexes entre cet anticorps et la galactoprotéine b3 ;
(d) de détection des immun-complexes formés entre ledit anticorps et ladite galactoprotéine b3 dans ledit échantillon biologique témoin ; et
(e) de comparaison des nombres d'immun-complexes détectés dans les étapes (b) et (d) et, ainsi, de détermination de la présence ou de l'absence de la tumeur.

29. Procédé suivant la revendication 28, dans lequel un groupe rapporteur est lié à un second anticorps capable de se lier à l'anticorps, et dans lequel l'étape de détection comprend
(a) l'élimination de pratiquement n'importe quelle quantité d'anticorps non lié,
(b) la mise en incubation avec le second anticorps dans des conditions et pendant un temps suffisants pour permettre la formation d'immun-complexes,
(c) l'élimination de pratiquement n'importe quelle quantité de second anticorps non lié, et
(d) la détection de la présence ou de l'absence du groupe rapporteur.

30. Procédé suivant la revendication 28, dans lequel un groupe rapporteur est lié à une molécule capable de se lier aux immun-complexes, et dans lequel l'étape de détection comprend
(a) la mise en incubation avec la molécule dans des conditions et pendant un temps suffisants pour permettre la liaison aux immun-complexes,
(b) l'élimination de pratiquement n'importe quelle molécule non liée, et
(c) la détection de la présence ou de l'absence du groupe rapporteur.

31. Procédé suivant la revendication 28, dans lequel un groupe rapporteur est lié à l'anticorps, et dans lequel l'étape de détection comprend l'élimination de pratiquement n'importe quel anticorps non lié, puis la détection de la présence ou de l'absence du groupe rapporteur.

32. Procédé suivant la revendication 29,30 ou 31, dans lequel le groupe rapporteur est choisi entre des radioisotopes, des fluorophores, des enzymes, des agents luminescents et des particules de colorants.
